(19) Europäisches Patentamt
European Patent Office
Office européen des brevets

(11) Veröffentlichungsnummer : **0 130 568**
**B1**

(12) # EUROPÄISCHE PATENTSCHRIFT

(45) Veröffentlichungstag der Patentschrift : **09.09.87**

(51) Int. Cl.[4] : **C 07 D301/19**

(21) Anmeldenummer : **84107462.8**

(22) Anmeldetag : **28.06.84**

(54) **Verfahren zur Herstellung von Oxiranen.**

(30) Priorität : **29.06.83 DE 3323329**

(43) Veröffentlichungstag der Anmeldung : **09.01.85 Patentblatt 85/02**

(45) Bekanntmachung des Hinweises auf die Patenterteilung : **09.09.87 Patentblatt 87/37**

(84) Benannte Vertragsstaaten : **BE DE FR GB IT NL**

(56) Entgegenhaltungen :
**EP-A- 0 005 100**
**DE-B- 2 436 817**
**US-A- 4 024 165**
**US-A- 4 177 196**
**US-A- 4 344 887**
**US-A- 4 389 529**

(73) Patentinhaber : **BASF Aktiengesellschaft**
**Carl-Bosch-Strasse 38**
**D-6700 Ludwigshafen (DE)**

(72) Erfinder : **Weitz, Hans-Martin, Dr.**
**Auf dem Koeppel 40**
**D-6702 Bad Duerkheim (DE)**
Erfinder : **Fischer, Rolf, Dr.**
**Bergstrasse 98**
**D-6900 Heidelberg (DE)**

**EP 0 130 568 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegenden Erfindung betrifft ein neues Verfahren zur Herstellung von Oxiranen durch Umsetzung von olefinisch ungesättigten Verbindungen mit organischen Peroxiden in flüssiger Phase sowie in Gegenwart von Epoxidierungskatalysatoren.

Aus zahlreichen Veröffentlichungen ist es allgemein bekannt, olefinisch ungesättigte Verbindungen mit organischen Peroxiden und Epoxidierungskatalysatoren in flüssiger Phase gemäß dem allgemeinen Reaktionsschema

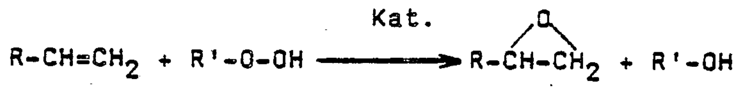


R, R′ = organische Reste

in die entsprechenden Oxirane und Hydroxylverbindungen zu überführen.

Hierbei ist grundsätzlich zwischen zwei verschiedenen verfahrenstechnischen Arbeitsrichtungen zu unterscheiden, nämlich derjenigen der homogenen und derjenigen der heterogenen Katalyse. In beiden Fällen dienen hierbei lösliche bzw. unlösliche Verbindungen von Metallen, vor allem des Wolframs, Molybdäns und Titans, als Epoxidierungskatalysatoren, die als Sauerstoffüberträger fungieren.

Reaktionsgemische, welche lösliche Epoxidierungskatalysatoren dieser Art enthalten, bieten verfahrentechnische Schwierigkeiten, da die Metallverbindungen bei der destillativen Aufarbeitung ausfallen oder desaktiviert werden. Die heterogene katalyse hat den Vorzug, daß sich der Katalysator hier leicht abtrennen läßt, jedoch stört eine derartige Operation prinzipiell den reibungslosen Fluß eines großtechnischen Verfahrens.

Es ist weiterhin bekannt, daß manche peroxidischen Verbindungen auch ohne Zuhilfenahme von Epoxidierungskatalysatoren zur Epoxidierung von olefinisch ungesättigten Verbindungen geeignet sind. Dies trifft z. B. für Ketonperoxide des Typs

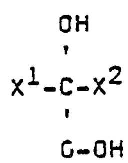

zu, wobei $X^1$ und $X^2$ Alkylgruppen mit hoher Fluor- und/oder Chlorsubstitution bedeuten.

Derartige Sauerstoffüberträger, die zum Zwecke der Epoxidierung beispielsweise aus der FR-PS-2 201 287 und der DE-AS-2 239 681 bekannt sind, müssen indes gesondert aus den entsprechenden Ketonen $X^1—CO—X^2$, insbesondere aus Hexafluoraceton, und Wasserstoffperoxid bereitet werden. Hierdurch leidet naturgemäß die Wirtschaftlichkeit eines technischen Epoxidierungsverfahrens, ganz abgesehen davon, daß das Arbeiten mit Wasserstoffperoxid, besonders wenn es in höheren Konzentrationen eingesetzt wird, nicht ungefährlich ist.

Der Erfindung lag die Aufgabe zugrunde, die Technik um ein Epoxidierungsverfahren in homogener flüssiger Phase zu bereichern, welches keine Aufarbeitungsschwierigkeiten wie im Falle löslicher Metallverbindungen bietet.

Demgemäß wurde ein Verfahren zur Herstellung von Oxiranen durch Umsetzung der entsprechenden olefinisch ungesättigten Verbindungen mit organischen Peroxiden in flüssiger Phase sowie in Gegenwart homogener Epoxidierungskatalysatoren gefunden, welches dadurch gekennzeichnet ist, daß man hierzu

ein Ketonperoxid (I) als organisches Peroxid, welches für sich allein nicht nennenswert mit der olefinisch ungesättigten Verbindung reagiert, und

als Epoxidierungskatalysator ein Keton der allgemeinen Formel II

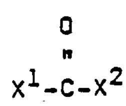 (II)

in der $X^1$ und $X^2$ Alkylgruppen mit hoher Fluor- und/oder Chlorsubstitution bedeuten, verwendet.

Als Ketonperoxide I eignen sich vor allem die Peroxide des Anthrachinons sowie von dessen Mono-, Di- und Tri—$C_1$—$C_6$-Alkylderivaten, die auf einfache Weise durch Luft- oder Sauerstoffoxidation der entsprechenden Anthrahydrochinone in organischer Lösung erhältlich sind :

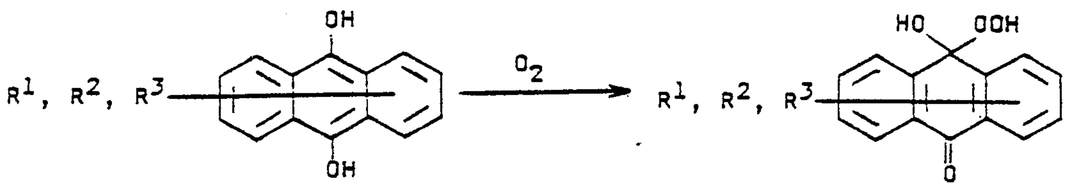


$R^1$, $R^2$, $R^3$ = H, $C_1$-$C_6$-Alkyl

Ob das Anthrachinonperoxid die vorstehend angegebene Struktur hat, ist nicht sicher, jedoch verhält es sich entsprechend dieser Struktur. Bei der Epoxidierungsreaktion geht das Peroxid in das Anthrachinon über, welches sodann in einem weiteren Verfahrensschritt wie üblich zum Hydrochinon hydriert werden kann, womit der Anthrachinonzyklus geschlossen ist.

Obwohl sich auch das unsubstituierte Anthrachinon für das erfindungsgemäße Verfahren eignet, ist den Alkylderivaten in der Regel der Vorzug zu geben, weil diese ein besseres Löslichkeitsverhalten haben. Bei den Alkylgruppen handelt es sich vor allem um die Ethyl-, i-Propyl-, -sec.-Butyl-, tert.-Butyl, sec.-Amyl und tert.-Amylgruppe, wobei ein bis drei dieser Substituenten an das Anthrachinongerüst gebunden sein können. Besonders zufriedenstellende Ergebnisse erzielt man mit dem leicht zugänglichen 2-tert.-Butylanthrachinon.

Neben den bevorzugten Anthrachinonperoxiden kommen ganz allgemein Ketonperoxide in Betracht, die für sich allein nicht oder nur in geringem Umfang zur Epoxidierung von olefinisch ungesättigten Verbindungen geeignet sind. Diese Peroxide leiten sich von aliphatischen und vor allem cycloaliphatischen Ketonen ab, die an den α-C-Atomen, bezogen auf die Oxogruppe, keine stark elektronegativen Substituenten wie Fluor oder Chlor tragen.

Die aliphatischen Ketone können 3 bis etwa 12 C-Atome aufweisen, und die cycloaliphatischen Ketone können 5 bis 8 Ringglieder haben. Bevorzugt wird hierunter das Cyclohexanonperoxid, da man das entstehende Cyclohexanon als Koppelprodukt verwerten kann, d. h. in diesem Falle kann die Stufe der Hydrierung des Ketons zum sekundären Alkohol entfallen. Die Ketonperoxide sind, wie das Anthrachinonperoxid, durch Luft- oder Sauerstoffoxidation der entsprechenden sekundären Alkohole erhältlich.

Bei den Epoxidierungskatalysatoren II handelt es sich vornehmlich um perfluorierte Ketone mit 3-8 C-Atomen, darunter vor allem um das leicht zugängliche, besonders wirksame hexafluoraceton, das auch in Form seiner Hydrate eingesetzt werden kann.

Weitere geeignete Verbindungen II sind die entsprechenden Chlorverbindungen oder auch gemischte Fluor/Chlor-Verbindungen. Allgemein gilt, daß die Wirksamkeit von II mit steigendem Halogenierungsgrad zunimmt — er soll mindestens etwa 80 % betragen — wobei Fluor jeweils den stärkeren Effekt erbringt. Weiterhin ist es vorteilhaft, wenn die α-C-Atome des Ketons zusammen mindestens drei Fluoratome tragen ; die restlichen Bindungen können dann auf Chlor und eventuell auch auf Wasserstoff entfallen.

Die Verbindungen II wirken mikrokinetisch als Sauerstoffüberträger, bleiben makrokinetisch indes unverändert. Theoretisch kommt man daher mit beliebig geringen Mengen an II aus. Da jedoch die Reaktionsgeschwindigkeit naturgemäß mit abnehmender Konzentration an II sinkt, wird es bevorzugt, pro Mol I mindestens 0,5 mol, vorzugsweise 0,5 bis 5 mol II zu verwenden. Zur weiteren verfahrenstechnischen Vereinfachung empfiehlt es sich darüber hinaus, II als Lösunsmittel zu verwenden, wobei der molare Überschuß von II über I dann etwa 5 bis 50 mol beträgt.

Als weitere Lösungsmittel kommen grundsätzlich alle organischen Flüssigkeiten in Betracht, die unter den Reaktionsbedingungen inert sind und die mit den Einsatzstoffen eine homogene flüssige Phase bilden.

Genannt seien beispielsweise $C_1$-$C_{12}$-Alkanole, Cyclohexanol, Benzylalkohol, Ester dieser Alkohole mit niederen Carbonsäuren wie Essigsäure oder auch beispielsweise mit Phthalsäure, Phosphorsäureester wie Trikresylphosphat, Kohlenwasserstoffe wie Benzol, Toluol, Xylol, Alkylnaphthaline, Tetrahydronaphthalin und Diphenyl, chlorierte Kohlenwasserstoffe wie Trichlorethylen, Ether wie 1,2-Dimethoxyethan, 1,2-Diethoxyethan, Diethylenglykoldimethylether und Anisol und cyclische Ether wie Dioxan und Tetrahydrofuran.

Die Menge des Lösungsmittels (einschließlich der Verbindungen II) soll so bemessen sein, daß alle Reaktionspartner eine homogene Phase bilden. Im allgemeinen liegt sie bei 2 bis 8 kg pro kg des dem Peroxid I zugrundeliegenden Ketons.

Das Verfahren ist prinzipiell von der Art der eingesetzten olefinisch ungesättigten Verbindung unabhängig und eignet sich daher grundsätzlich zur Herstellung beliebiger Oxirane. Bedingt oder nicht geeignet sind, wie sich allerdings von selbst versteht, lediglich solche olefinisch ungesättigten Verbindungen, die Substituenten tragen, welche in größerem Umfang zu Nebenreaktionen Anlaß geben, also etwa saure oder basische Gruppen.

Die olefinisch ungesättigten Verbindungen können des weiteren auch zwei oder mehrere -C = C-Gruppierungen enthalten ; in diesem Falle entstehen die entsprechenden Bis- und Polyoxirane.

Unter den olefinisch ungesättigten Verbindungen sind in erster Linie die $C_2$-$C_{20}$-Olefine zu nennen. Hierbei können die $C_4$-$C_{20}$-Olefine unverzweigt oder verzweigt sein, und die Doppelbindung kann inneständig oder endständig sein. Besondere Bedeutung hat das Propylen als Vorstufe für das im großtechnischen Maßstab benötigte Propylenoxid.

Weiterhin kommen in Betracht:

Cycloolefine wie Cyclopenten, Cyclohexen, Cycloocten und Cyclododecen
Bisolefine wie Buta-1,3-dien, Isopren und Hexa-1,5-dien
araliphatische Olefine wie Styrol und Stilben
Vinylalkylether wie Vinylethylether und Vinylester wie Vinylacetat
Allylalkylether wie Allylmethylether und Allylester wie Allylacetat und
Acrylsäure und Methacrylsäure

Allgemein können die olefinisch ungesättigten Verbindungen Substituenten wie Halogen, die Nitrogruppe, die Cyangruppe, Alkoxygruppen und Carbalkoxygruppen tragen. Im Falle von Hydroxylgruppen sowie von Oxogruppen in Aldehyd- oder Ketonfunktion wäre durch Vorversuche festzustellen, ob und inwieweit diese unter den Reaktionsbedingungen zu Nebenreaktionen Anlaß geben.

Olefinisch ungesättigte Verbindung und Peroxid I werden zweckmäßigerweise im Molverhältnis von 0,5 : 1 bis 10 : 1, vorzugsweise von 1 : 1 bis 5 : 1 eingesetzt. Man nimmt die Umsetzung im allgemeinen bei 20 bis 150, vorzugsweise 40-120 °C sowie unter Normaldruck vor. Höherer Druck, etwa bis 50 bar, ist möglich, ist in der Regel jedoch nicht erforderlich, es sei denn, die olefinisch ungesättigte Verbindung wäre bei der Reaktionstemperatur gasförmig.

Das erfindungsgemäße Verfahren eignet sich besonders gut für die kontinuierliche Arbeitsweise, da keine Feststoffe abgetrennt werden müssen und da auch keine Störungen durch ausfallende Feststoffe zu befürchten sind.

Der Ablauf eines kontinuierlichen Verfahrens kann sich beispielsweise wie folgt gestalten:

a) Hydrierung des dem Peroxid I zugrundeliegenden Ketons in einem Hydrierreaktor mit Wasserstoff und einem Hydrierkatalysator in an sich bekannter Weise, vorzugsweise in II als Lösungsmittel unter Bedingungen, unter denen II nicht ebenfalls nennenswert hydriert wird; die Hydrierung von II schadet andererseits nicht, da die Ketonform in der folgenden Stufe zurückgebildet wird; Überführung des Reaktionsgemisches in

b) eine Blasensäule, Füllkörper- oder Bodenkolonne, wo es mit Sauerstoff oder einem Sauerstoff enthaltenden Gasgemisch, beispielsweise Luft, in Kontakt gebracht wird, wobei sich das peroxid I bildet; Überführung dieses Reaktionsgemisches in

c) eine Füllkörper- oder Bodenkolonne und Umsetzung mit der hier hinein eingeleiteten olefinisch ungesättigten Verbindung;

d) destillative Fraktionierung des in Stufe (c) erhaltenen Reaktionsgemisches in das Oxiran, eine Fraktion aus olefinisch ungesättigter Verbindung und gegebenenfalls II sowie ein Fraktion aus dem dem Peroxid I zugrundeliegenden Keton und gegebenenfalls I und

e) Rückführung der letztgenannten Fraktionen in Stufe (c) bzw. Stufe (a).

Bei Mitverwendung eines weiteren Lösungsmittels, also wenn nicht nur II als Lösungsmittel dient, ändert sich an dem Verfahrensgang prinzipiell nichts. Will man das dem Peroxid I zugrundeliegende Keton als Koppelprodukt gewinnen, entfällt die Hydrierstufe. Ferner gilt allgemein, daß die Stufen (b) und (c) in einem Verfahrensschritt zusammengefaßt werden können.

Das erfindungsgemäße Verfahren liefert in der Regel Oxiranausbeuten bis zwischen 90 und 99 %, bezogen auf das eingesetzte Peroxid I.

## Beispiele 1-15

Herstellung verschiedener Oxirane

Lösungen aus je 73 g iso-Butylacetat und 5,96 g (20 mmol) 2-tert.-Butylanthrachinonperoxid, die durch katalytische Hydrierung der Anthrachinonlösungen und anschließende Oxidation des Anthrahydrochinons mit Sauerstoff hergestellt worden waren, wurden unter verschiedenen Bedingungen mit Oct-1-en bzw. mit Cyclohexen und Styrol in Gegenwart von Hexafluoraceton-Sesquihydrat (HFA.1,5$H_2O$) umgesetzt. Im einzelnen sind die Bedingungen und die gaschromatographisch ermittelten Ergebnisse der Versuche der folgenden Tabelle zu entnehmen.

## Beispiel 16

Eine Lösung aus 2,6 g (20 mmol) Cyclohexanonperoxid und 50 g Cyclohexanol, die durch Sauerstoffoxidation von Cyclohexanol hergestellt worden war, wurde mit 22,4 g (0,2 mol) Oct-1-en und 3,86 g (20 mmol) Hexafluoraceton-Sesquihydrat versetzt und 24 h bei 60 °C gerührt. Die auf den Peroxidumsatz von 25 % bezogene Octenoxidselektivität betrug 64 %.

(Siehe Tabelle Seite 5 f.)

Tabelle, Beispiele 1-15

| Beisp. | Olefin | Molverh. Olef.:Peroxid | $HFA.1,5H_2O$ bez.auf Peroxid mol% | Temp. °C | Reaktionszeit h | Peroxid-Umsatz % | Oxiran-Selektivität +) % |
|---|---|---|---|---|---|---|---|
| 1 | Oct-1-en | 10:1 | 100 | 90 | 4 | 93 | 99 |
| 2 | " | 1:1 | 100 | 90 | 22 | 92 | 41 |
| 3 | " | 5:1 | 100 | 90 | 6 | 95 | 70 |
| 4 | " | 10:1 | 5 | 90 | 24 | 82 | 73 |
| 5 | " | 10:1 | 10 | 90 | 24 | 95 | 69 |
| 6 | " | 10:1 | 50 | 90 | 6 | 90 | 88 |
| 7 | " | 10:1 | 200 | 90 | 4 | 99 | 99 |
| 8 | " | 10:1 | 1000 | 90 | 1,5 | 98 | 87 |
| 9 | " | 10:1 | 100 | 60 | 24 | 74 | 73 |
| 10 | " | 10:1 | 100 | 90 | 4 | 93 | 98 |
| 11 | " | 10:1 | 100 | 115 | 0,7 | 99 | 76 |
| 12 | " | 10:1 | 5 | 115 | 6 | 98 | 64 |
| 13 | " | 10:1 | 50 | 115 | 1,5 | 99 | 80 |
| 14 | Cyclohexen | 10:1 | 100 | 90 | 1 | 97 | 98 |
| 15 | Styrol | 10:1 | 100 | 90 | 1,5 | 97 | 98 |

+) bezogen auf umgesetztes Peroxid

**Patentansprüche**

1. Verfahren zur Herstellung von Oxiranen durch Umsetzung der entsprechenden olefinisch ungesättigten Verbindungen mit organischen Peroxiden in flüssiger Phase sowie in Gegenwart homogener Epoxidierungskatalysatoren, dadurch gekennzeichnet, daß man hierzu

ein Ketonperoxid (I) als organisches Peroxid, welches für sich allein nicht nennenswert mit der olefinisch ungesättigten Verbindung reagiert, und

als Epoxidierungskatalysator ein Keton der allgemeinen Formel II,

$$X^1-\overset{\overset{\displaystyle O}{\|}}{C}-X^2 \qquad (II)$$

in der $X^1$ und $X^2$ Alkylgruppen mit hoher Fluor- und/oder Chlorsubstitution bedeuten, verwendet.

2. Verfahren nach Anspruch 1, dadurch gekennzeichnet, daß man als Ketonperoxid (I) ein Anthrachinonperoxid verwendet.

3. Verfahren nach Anspruch 2, dadurch gekennzeichnet, daß man als Anthrachinonperoxid 2-tert.-Butylanthrachinonperoxid verwendet.

4. Verfahren nach den Ansprüchen 1 bis 3, dadurch gekennzeichnet, daß man als Verbindung II Hexafluoraceton verwendet.

**Claims**

1. A process for the preparation of an oxirane by reacting the corresponding olefinicaly unsaturated compound with an organic peroxide in the liquid phase and in the presence of a homogeneous epoxidation catalyst, wherein

a ketone-peroxide (I) is used as the organic peroxide, which itself does not react significantly with the olefinically unsaturated compound, and

a ketone of the general formula II

$$X^1-\overset{\overset{O}{\|}}{C}-X^2 \quad (II)$$

where $X^1$ and $X^2$ are each alkyl having a high degree of substitution by fluorine and/or chlorine, is used as the epoxidation catalyst.

2. A process as claimed in claim 1, wherein an anthraquinone peroxide is used as the ketone-peroxide (I).

3. A process as claimed in claim 2, wherein 2-tert.-butylanthraquinone peroxide is used as the anthraquinone peroxide.

4. A process as claimed in claims 1 to 3, wherein hexafluoroacetone is used as compound II.

**Revendications**

1. Procédé de préparation d'oxirannes par réaction des composés oléfiniquement insaturés correspondants avec des peroxydes organiques, en phase liquide et en présence de catalyseurs d'époxydation homogènes, caractérisé en ce qu'on utilise à cet effet

en tant que peroxyde organique, un peroxyde de cétone (I) qui, à lui seul, ne réagit pas de façon appréciable avec le composé oléfiniquement insaturé et

en tant que catalyseur d'époxydation, une cétone de formule générale II

$$X^1-\overset{\overset{O}{\|}}{C}-X^2 \quad (II)$$

dans laquelle $X^1$ et $X^2$ représentent des groupements alkyle à forte substitution par le fluor et/ou le chlore.

2. Procédé selon la revendication 1, caractérisé en ce qu'on utilise, comme peroxyde de cétone (I), un peroxyde d'anthraquinone.

3. Procédé selon la revendication 2, caractérisé en ce qu'on utilise, comme peroxyde d'anthraquinone, le peroxyde de 2-tert.-butylanthraquinone.

4. Procédé selon l'une quelconque des revendications 1 à 3, caractérisé en ce qu'on utilise, en tant que composé II, l'hexafluoracétone.